# EUROPEAN PATENT APPLICATION

(11) **EP 4 410 447 A1**
(43) Date of publication of application: **07.08.2024**
(21) Application number: 23154841.3
(22) Date of filing: 03.02.2023
(51) Int. Cl.: B21D 39/04, B21D 26/14, A61M 15/00, B21D 51/26, B65D 83/38, B65D 83/54

(54) **METHOD FOR PRODUCING A MEDICAMENT CANISTER FOR METERED DOSE INHALERS**

(71) Applicant: Presspart GmbH & Co. KG, 34431 Marsberg (DE)
(72) Inventor: Ramspott, Janis, 34431 Marsberg (DE); Giebel, Andreas, 34431 Marsberg (DE)
(74) Representative: Uexküll & Stolberg

(57) **Abstract**

Provided is a method for producing a gas tight medicament canister (1) for metered dose inhalers, the medicament canister (1) comprising a metal or plastic container (2) and a valve cap (3). The method comprises the steps of:
a) providing the container (2) with an interior cavity (4) defined by a circumferential side wall (5) extending from a bottom wall (6) to an open upper circular end (7) of the container (2), the side wall (5) at the open upper end (7) of the container (2) having a first diameter (d1);
b) providing the valve cap (3) having a metering valve (8) and a cap housing (9), in which the metering valve (8) is mounted, the cap housing (9) having a tubular wall portion (10) forming an open lower end (11) of the cap housing (9), wherein the tubular wall portion (10) has a second diameter (d2) which is slightly larger than the first diameter (d1) at the open upper end (7) of the container (2), such that, upon overlap of the open lower end (11) of the cap housing (9) and the open upper end (7) of the container (2), the tubular wall portion (10) encloses the side wall (5) of the container (2);
c) positioning the lower end (11) of the cap housing (9) over the upper end (7) of the container (2) such that at least a portion of the tubular wall portion (10) of the cap housing (9) overlaps with a portion of the side wall (5) of the container (2), thereby forming an overlap region (12) between the lower end (11) of the cap housing (9) and the open upper end (7) of the container (2);
d) applying one or more magnetic field pulses to the overlap region (12) between the lower end (11) of the cap housing (9) and the open upper end (7) of the container (2), thereby generating a magnetic pressure force which acts on the portion of the tubular wall portion (10) of the cap housing (9) and/or the portion of the side wall (5) of the container (2) in a direction substantially perpendicular to a circumference of the overlap region (12), thereby joining the portion of the tubular wall portion (10) and the portion of the tubular side wall (5) in a gas tight manner.

## Description

### FIELD OF THE INVENTION

The invention relates to a method for producing medicament canisters which are suitable for storing a medicament and a propellant under pressure, and which are useful in metered dose inhalers.

### BACKGROUND OF THE INVENTION

Metered dose inhalers, such as pressurized metered dose inhalers (pMDI), are medication delivery devices which deliver a pharmaceutical formulation including one or more pharmaceutical active compounds to human or other mammalian patients. Typically, the pharmaceutical formulation is delivered by the pMDIs in unit doses in the form of an aerosol. Each actuation of the MDIs delivers one unit dose. The unit dose is expelled by the MDIs and is taken into the body of the patient on inhalation via the nose or mouth. The pharmaceutical formulation is delivered to or via the respiratory tract, notably to the lungs, of the patient on inhalation. Metered dose inhalers are typically used for the treatment of respiratory and nasal infections and disorders, including asthma, respiratory tract infections, obstructive lung disease, inflammatory lung disease and chronic obstructive pulmonary disease. Asthma treatment is a particularly common use of MDIs.

The medicament is typically contained in a medicament canister, and a propellant, a low boiling point liquid or liquified gas, is usually formulated with the respective medicament and disposed under pressure in the canister. The canister must be capable of withstanding the vapor pressure of the propellant, and is usually a metal container, for example a stainless steel container or an aluminum container, which is closed by a valve cap comprising a metering valve. The canister, however, may also be a plastic container.

For use, the canister is arranged in an inhaler body which receives the canister and which provides a channel for guiding the formulation of medicament and propellant expelled from the metering valve into the mouth or nose of the patient.

Metering valves comprising a metering chamber are designed to deliver a predetermined quantity of the medicament to be administered per actuation, and are affixed to a container body by crimping. A valve stem extends from the metering valve and acts as a conduit to pass the metered dose into a nozzle block located in an inhaler body which further guides the formulation of medicament and propellant expelled from the metering valve into the mouth or nose of the patient.

The combination of inhaler body with a medicament canister comprising a container filed with the propellant and medicament and being closed by a metering valve is commonly referred-to as metered dose inhaler (MDI).

In order to maintain the propellant in the liquid state, a considerable pressure must be maintained within the interior of the canister. Consequently, the canister must be capable of safely withstanding such elevated pressures. Specifically, according to ICAO regulations the burst pressure of the canister must be at least 220 psi, i.e. 1.52 MPa.

As a safety measure, the bottom wall of the container is typically formed into an inwardly domed shape which inverts into an outwardly domed shape upon exceeding a certain pressure somewhat below the admissible 1.52 MPa. Such embodiment is e.g. disclosed in EP 2 711 309 A1 or EP 3 356 256 B1, which are each incorporated herein by reference in their entirety.

On the other side of the canister, the metering valve is sealed to the container with a cap that includes the metering valve. The seal is commonly effected by mechanically crimping the valve cap onto a neck of the container, as described e.g. in US 2002/0152628 A1, also incorporated herein by reference in its entirety. The canister is then charged, many times, through the valve stem with the medicament and/or the propellant.

As known to those skilled in the art, the quality of the crimping process by which the valve cap is sealed onto the container is of utmost criticality. The slightest leak will render the canister commercially valueless. By the time the defective canister has been distributed to the patient, most or all of the propellant will have escaped the confines of the canister and the canister rendered inoperative.

Usually a rubber gasket is used to ensure integrity of the seal. However, even canisters having a properly crimped valve cap sealed onto the container have shown not to be entirely gas tight. Leakage paths have still been identified between the container and the mechanically crimped valve cap. Poor gasket quality has often been identified as the root cause.

It is therefore an object of the present invention to provide a method of manufacturing a medicament canister with a gas tight connection between the container and the valve cap, the canister being useful for metered dose inhalers. Moreover, it is an object of the present invention to provide a corresponding medicament canister.

### SUMMARY OF THE INVENTION

The object is achieved with the method as defined in present claim 1. Preferred embodiments are illustrated in the appending sub-claims.

According to the invention, there is provided a method of manufacturing a medicament canister for metered dose inhalers, the medicament canister comprising a metal or plastic container and a valve cap, the method comprising the steps of
- providing the container with an interior cavity defined by a circumferential side wall extending from a bottom wall to an open upper circular end of the container, the side wall at the open upper end of the container having a first diameter,
- providing the valve cap having a metering valve and a cap housing, in which the metering valve is mounted, the cap housing having a tubular wall portion forming an open lower end of the cap housing, wherein the tubular wall portion has a second diameter which is slightly larger than the first diameter at the open upper end of the container, such that, upon overlap of the open lower end of the cap housing and the open upper end of the container, the tubular wall portion encloses the side wall of the container,
- positioning the lower end of the cap housing over the upper end of the container such that at least a portion of the tubular wall portion of the cap housing overlaps with a portion of the side wall of the container, thereby forming an overlap region between the lower end of the cap housing and the open upper end of the container,
- applying one or more magnetic field pulses to the overlap region between the lower end of the cap housing and the open upper end of the container, thereby generating a magnetic pressure force which acts on the portion of the tubular wall portion of the cap housing and/or the portion of the side wall of the container in a direction substantially perpendicular to a circumference of the overlap region, thereby joining the portion of the tubular wall portion and the portion of the tubular side wall in a gas tight manner.

According to the invention one or more magnetic field pulses to the overlap region between the lower end of the cap housing and the open upper end of the container are used to create a gas-tight connection between the valve cap and the container. A gasket between the tubular side wall of the container and the cap housing of the valve cap is not required to provide a gas-tight connection between container and valve cap thereby reducing costs and assembly time of the canister. However, a gasket may be installed as an additional safety measure, in case customers demand so.

In connection with the present invention the technique of generating one or more magnetic field pulses to create a gas-tight connection is referred to as electromagnetic pulse technology. This technology includes electromagnetic pulse welding and electromagnetic pulse crimping. The main difference between electromagnetic pulse welding and electromagnetic pulse crimping is the energy input used to create the field pulses. Electromagnetic pulse welding uses a higher energy input which results in a substance-to-substance bond. Compared to electromagnetic pulse welding, electromagnetic pulse crimping uses a lower energy input and provides a mechanical connection between the valve cap and the container. Besides the energy input further parameters such as the material combination of the parts to be connected, the wall thickness of the parts at the area to be connected, the distance between the parts at the area to be connected and the material properties may influence whether the parts to be connected are joined by electromagnetic pulse welding or electromagnetic pulse crimping. However, electromagnetic pulse welding and electromagnetic pulse crimping both provide a gas-tight connection between the container and the valve cap.

Electromagnetic pulse welding and crimping do not generate particulates, which is why these techniques may be also used in sterile environments. Moreover, electromagnetic pulse welding and crimping is a "cold" joining process such that no heat affected zone is created and the materials of the container and the valve cap do not lose their properties. The absence of heat and the solid-state character of the process enables for example to join dissimilar materials. The technique of electromagnetic pulse welding is described in T. Sapanathan et al., "Magnetic Pulse Welding: An Innovative Joining Technology for Similar and Dissimilar Metal Pairs" in Joining Technologies, M. Ishak (Ed.), 2016, pages 243-273, which is incorporated herein by reference.

In connection with the present invention the term "magnetic pressure force" is to be understood as a force caused by a gradient in field strength of a magnetic field. Any magnetic field has an associated magnetic pressure contained by the boundary conditions on the field. A gradient in field strength causes a force due to the magnetic pressure gradient called the magnetic pressure force.

In an embodiment of the invention the difference between the second diameter of the tubular wall portion and the first diameter at the open upper end of the container lies in the range of about 0.2 mm to 8 mm, preferably of about 1 mm to 5 mm, more preferably of about 1.5 mm to 3 mm, most preferably is about 2 mm in the step of providing the valve cap. The above differences between the first and second diameters allow an easy positioning of the cap housing over the upper end of the container without canting. Moreover, the above differences facilitate the generation of a magnetic pressure force which is able to deform and join the portion of the tubular wall portion of the cap housing and the portion of the tubular side wall.

In an embodiment of the invention the side wall extending from the bottom wall to the open upper circular end of the container has a tubular shape in the step of providing the container and at least a portion of the tubular wall portion of the cap housing overlaps with a portion of the tubular side wall of the container in the step of positioning the lower end of the cap housing over the upper end of the container. A container having a tubular sidewall is cost efficient and easy to manufacture.

In an embodiment of the invention the tubular side wall comprises an annular flange at the open upper end of the container in the step of providing the container, the flange extending in a radial outward direction, and at least a portion of the tubular wall portion of the cap housing overlaps with the flange and a portion of the tubular side wall of the container in the step of positioning the lower end of the cap housing over the upper end of the container. Upon deformation of the portion of the tubular wall portion of the cap housing cap housing due to the magnetic pressure force, the portion of the tubular wall portion engages behind the surface of the flange which points towards the bottom wall of the container. This ensures the required contact surface to seal the tubular wall portion of the cap housing against the flange of the container. Preferably, the length of the tubular wall portion of the cap housing is chosen such that the tubular wall portion upon deformation also abuts the tubular sidewall of the container. This strengthens the connection between the cap housing and the container.

In an embodiment of the invention the side wall comprises a reduced diameter section in the step of providing the container and the tubular wall portion of the cap housing is deformed to abut the side wall at the reduced diameter section in the step of applying one or more magnetic field pulses. The reduced diameter section may be provided by a concave portion of the sidewall connecting the upper open end with the tubular sidewall of the container, the tubular sidewall extending from the bottom wall of the container to the reduced diameter section. Alternatively, the reduced diameter section may be provided by a concave portion of the sidewall which connects an upper tubular sidewall portion extending from the concave portion to the open upper end of the container with the tubular sidewall of the container, the tubular sidewall extending from the bottom wall of the container to the reduced diameter section.

In an embodiment of the invention the step of positioning the lower end of the cap housing over the upper end of the container includes the inserting of the container and the valve cap into an apparatus configured to generate the one or more magnetic field pulses, the apparatus comprising:
- a coil surrounding the overlap region between the lower end of the cap housing and the open upper end of the container,
- a field shaper positioned between the coil and the overlap region, the field shaper being configured to concentrate the magnetic pressure force on the overlap region and
- a capacitor connected to the coil.

For the effective use of magnetic pressure forces during forming, it is favorable if the distance from the coil to the overlap region of the cap housing and the container is as small as possible. Moreover, it may be desirable to use the apparatus for different container and valve cap sizes, the container and valve caps differing in their diameter. In order to address these issues, a field shaper is used which is configured to concentrate the electromagnetic force effect on the overlap region. Preferably, the field shaper is isolated from the coil. Optionally, the field shaper has a cross sectional area along its longitudinal axis, wherein the longitudinal axis of the field shaper coincides with a longitudinal axis of the coil and a longitudinal axis of the container. The cross sectional area of the field shaper tapers towards the overlap region between the lower end of the cap housing and the open upper end of the container.

In an embodiment of the invention the field shaper is designed as two parts each forming a half shell. Due to the skin effect the current induced by the coil only flows on the surfaces of the field shaper. The field shaper forms two coupled coils (one on the outer diameter/and one on the inner diameter of the field shaper), is preferably made of copper as this material provides a high electrical conductivity and is preferably slotted at least once along the longitudinal axis of the coil so that the induced current can reach the inner diameter.

In an embodiment of the invention the apparatus further comprises a centering guide adapted to center the container and the valve cap in the field shaper and the coil. This allows a simplified insertion of the container and the valve cap into the apparatus. Moreover, it is ensured that the overlap region between the lower end of the cap housing and the open upper circular end of the container is correctly positioned relative to the field shaper and the coil.

In an embodiment of the invention the step of applying one or more magnetic field pulses to the overlap region includes the charging of the capacitor with a predetermined amount of energy, the instantaneously releasing of the energy into the coil surrounding the overlap region, thereby generating the magnetic pressure force and causing the portion of the tubular wall portion of the cap housing to impact onto the portion of the tubular sidewall of the container. In electromagnetic pulse welding and electromagnetic pulse crimping, electromagnetic forces are used to impact two materials against each other at high speed. A power supply is used to charge a capacitor. Optionally, a capacitor bank instead of a capacitor may be used depending on the required amount of energy. When the predetermined amount of energy is stored in the capacitor, it is instantaneously released into a coil. The discharge current induces a strong transient magnetic field inside the coil, thereby generating a magnetic field pulse. The transient magnetic field in turn induces eddy currents in the portion of the tubular wall portion of the cap housing. These eddy currents prevent the magnetic field to diffuse through the tubular wall portion of the cap housing and cause a difference in magnitude of the magnetic field on the portion of the tubular wall portion of the cap housing and the portion of the side wall of the container. The difference generates a magnetic pressure force, which causes the portion of the tubular wall portion of the cap housing to impact with the portion of the tubular side wall of the container. When using a predetermined amount of energy which results in electromagnetic pulse welding the collision between both portions causes bonding through several bonding mechanisms. Bonding between materials is created when the distance between their atoms becomes smaller than the range of their mutual attractive forces. In that case, electrons are shared between the two materials and an intermetallic phase can be formed. In connection with the present invention such bonding mechanisms are referred to by the term substance-to-substance bond.

In another embodiment of the invention the predetermined amount of energy stored in the capacitor is at most 0,9 kJ, preferably at most 0,75 kJ, more preferably at most 0,45 kJ, most preferably at most 0,3 kJ. The higher the amount of energy the stronger the forming behavior but also the risk of deforming the canister. However, the amount of energy used to charge the capacitor bank should be chosen such that a sufficiently tight form fit between the lower end of the cap housing and the open upper end of the container is achieved. In connection with the present invention it has been found that at most 0,45 kJ, such as between 0,35 kJ and 0,45 kJ may be sufficient to achieve a reliable gas tight connection between the cap housing and the container but at the same time to cause only minimal deformation of the canister. The amount of energy used defines whether there will be a substance-to-substance bond between the valve cap and the container by electromagnetic pulse welding or whether there will be a mechanical connection between the both by electromagnetic pulse crimping. Electromagnetic pulse welding uses a higher amount of energy used to charge the capacitor bank than electromagnetic pulse crimping. Besides the energy input further parameters such as the material combination of the parts to be connected, the wall thickness of the parts at the area to be connected, the distance between the parts at the area to be connected and the material properties may influence whether the parts to be connected are joined by electromagnetic pulse welding or electromagnetic pulse crimping.

In another embodiment of the invention no gasket is inserted between the tubular side wall of the container and the cap housing of the valve cap. Avoiding an additional gasket reduces production costs and assembly time.

In another embodiment of the invention the container and/or the cap housing are made of stainless steel or aluminum. Preferably, the cap housing and the container are made from the same material. Stainless steel and aluminum are for example suitable materials for being joined by the method of the invention. Optionally, the cap housing and the container are made from different materials. The process of electromagnetic pulse welding allows the joining of different materials as long as they provide electrical conductivity. As such, stainless steel and aluminum are well suited for being joined via electromagnetic pulse welding. However, for the process of electromagnetic crimping it is sufficient, if one the used materials provides electrical conductivity. For example the container may be formed of plastic, whereas the cap housing is made of stainless steel or aluminum.

In another embodiment of the invention the method further comprises the step of filing the medicament canister with propellant and medicament under pressure via the metering valve or filling the medicament container with medicament prior to joining the container and the cap housing of the valve cap and filling the canister with propellant thereafter via the metering valve. Preferably, the medicament canister is provided to a filling station configured to fill the canister with propellant and medicament under pressure via the metering valve and/or configured to fill the medicament container with medicament prior to joining the container and the cap housing of the valve cap and to fill the canister with propellant thereafter via the metering valve. The medicament canister may be provided to the filling station subsequent to the electromagnetic pulse welding or crimping.

The above object is also achieved with a medicament canister as defined in claim 14. A preferred embodiment of the medicament canister is provided in dependent claim 15.

According to the invention the medicament canister is obtainable by the method as described above. The medicament canister comprises an annular gas tight connection between the container and the valve cap in the overlap region. By use of electromagnetic pulse technology including electromagnetic pulse welding and electromagnetic pulse crimping a gas tight connection between the portion of the tubular wall portion of the cap housing and the portion of the tubular side wall of the container can be generated. Compared to the traditional joining of the cap housing and the container by crimping, electromagnetic pulse welding and electromagnetic pulse crimping reduces the number of leaking medicament canisters and thus reduces scrap in the production process. The risk of slow leaking cans due to an improper connection of the cap housing and the container is minimized.

In an embodiment of the invention there is no gasket between the tubular side wall of the container and the cap housing of the valve cap. Avoiding an additional gasket reduces production costs and assembly time.

### BRIEF DESCRIPTION OF THE FIGURES

The invention will now be described in connection with an exemplary embodiment shown in the Figures in which:
- Figure 1: shows a cross-sectional view of a medicament canister according to the present invention,
- Figure 1a: shows a cross-sectional view of a medicament canister as known from the prior art,
- Figure 2: shows a cross-sectional view of a second embodiment of a medicament canister according to the present invention,
- Figure 3: shows a cross-sectional view of a third embodiment of a medicament canister according to the present invention,
- Figure 4: shows a cross-sectional view of a fourth embodiment of a medicament canister according to the present invention and
- Figure 5: shows a cross-sectional view of an apparatus for generating one or more magnetic field pulses and the medicament canister of Figure 1 inserted into the apparatus.

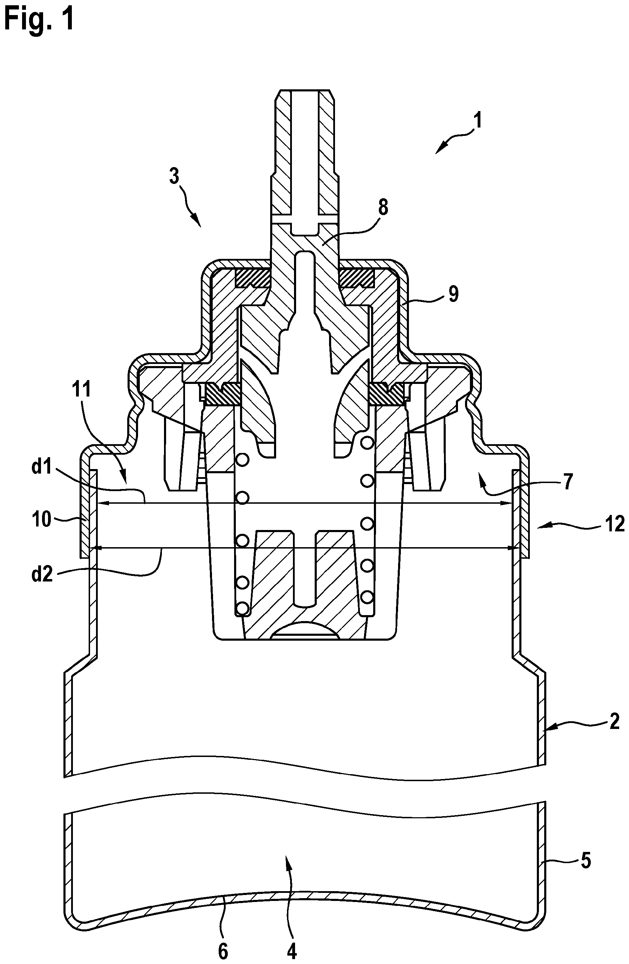

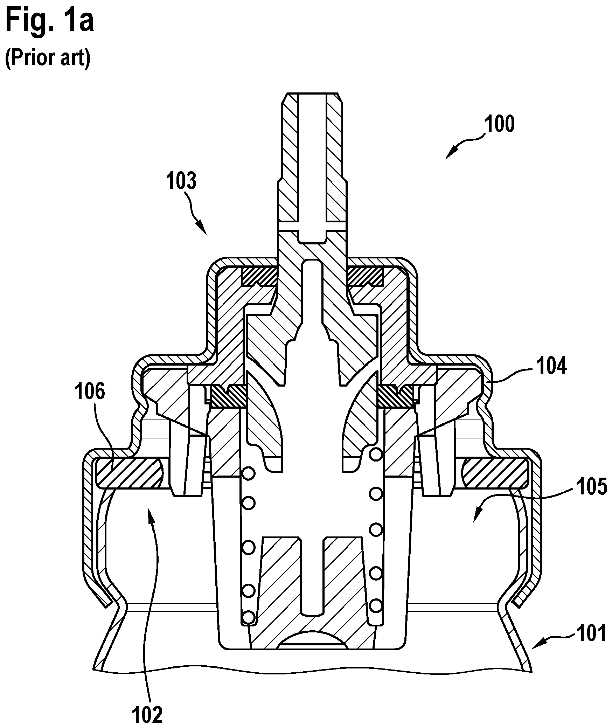

Figure 1 shows a medicament canister 1 for metered dose inhalers (not shown). The medicament canister 1 comprises a metal or plastic container 2 and a valve cap 3. The valve cap 3 and the container 2, when made of metal, are made of stainless steel or aluminum. The valve cap 3 may also be formed of metal or plastic.

The container 2 has an interior cavity 4 defined by a circumferential side wall 5 having a tubular shape, the sidewall 5 extending from a bottom wall 6 to an open upper circular end 7 of the container 2. The tubular side wall 5 at the open upper end 7 has a first (outer) diameter d1.

The valve cap 3 has a metering valve 8 and a cap housing 9, in which the metering valve 8 is mounted. The cap housing 9 has a tubular wall portion 10 forming an open lower end 11 of the cap housing 9. The tubular wall portion 10 has a second (inner) diameter d2 which is slightly larger than the first diameter d1 at the open upper end 7 of the container 2, such that, the tubular wall portion 10 encloses the tubular side wall 5 of the container 2. The difference between the second diameter d2 of the tubular wall portion 10 and the first diameter d1 at the open upper end 7 of the container 2 lies in the range of about 0.2 mm to 8 mm, preferably of about 1 mm to 5 mm, more preferably of about 1.5 mm to 3 mm, most preferably is about 2 mm.

In Figure 1 the cap housing is positioned relative to the container 2 in such a way that a portion of the tubular wall portion 10 of the cap housing 9 overlaps with a portion of the tubular side wall 5 of the container 2, thereby forming an overlap region 12 between the lower end 11 of the cap housing 9 and the open upper end 7 of the container 2.

The medicament canister 1 comprises an annular gas tight connection between the container 2 and the valve cap 3 in the overlap region 12 formed by electromagnetic pulse technology which includes electromagnetic pulse welding and electromagnetic pulse crimping. There is no gasket between the tubular side wall 10 of the container 2 and the cap housing 9 of the valve cap 3.

Figure 1a shows a medicament canister 100 as known from the prior art. The medicament canister 100 comprises a metal container 101 with an open upper end 102 and a valve cap 103. The valve cap 103 has a cap housing 104 with an open lower end 105.

The cap housing 104 and the metal container 101 are joined by mechanical crimping, wherein a portion of the open lower end 105 of the cap housing 104 is crimped onto a portion of the open upper end 102 of the container 101. The mechanical crimping does not form a gas-tight connection as a small gap between the crimped portions remains.

In order to seal this gap and to provide a gas-tight connection between the container 101 and the cap housing 104 a gasket 106 is positioned between the open upper end 102 of the metal container 101 and the cap housing.

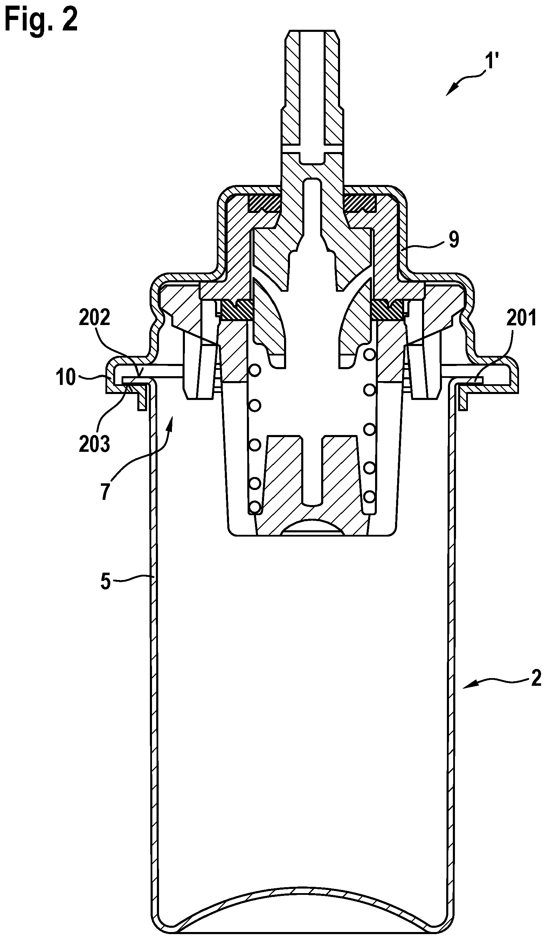

Figure 2 shows a second embodiment of a medicament canister 1 according to the present invention. The medicament canister 1' differs from the one shown in Figure 1 in that the tubular side wall 5 of the container 2 comprises an annular flange 201 at the open upper end 7 of the container 2. The flange 201 extends in a radial outward direction and comprises an upper side 202 and a lower side 203. A portion of the tubular wall portion 10 of the cap housing 9 overlaps with the flange 201 and preferably overlaps with a portion of the tubular side wall 5 of the container 2. In detail, the portion of the tubular wall portion 10 of the cap housing 9 encompasses the upper side 202 and the lower side 203 of the flange 201 and preferably further abuts a portion of the tubular side wall 5 of the container 2.

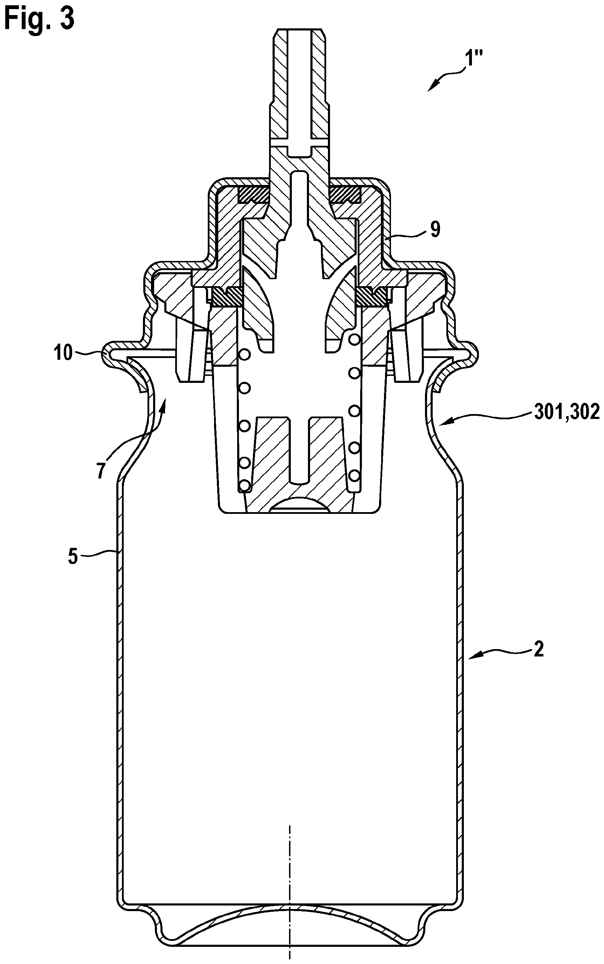

Figure 3 shows a third embodiment of a medicament canister 1 according to the present invention. The medicament canister 1'' differs from the one shown in Figure 1 in that the side wall 5 of the container 2 comprises a reduced diameter section 301. The reduced diameter section 301 is formed by a concave portion 302 of the sidewall 5 connecting the upper open end 7 with the tubular sidewall 5 of the container 2. The tubular wall portion 10 of the cap housing 9 is deformed by electromagnetic pulse welding or crimping to abut the side wall 5 at the concave portion 302.

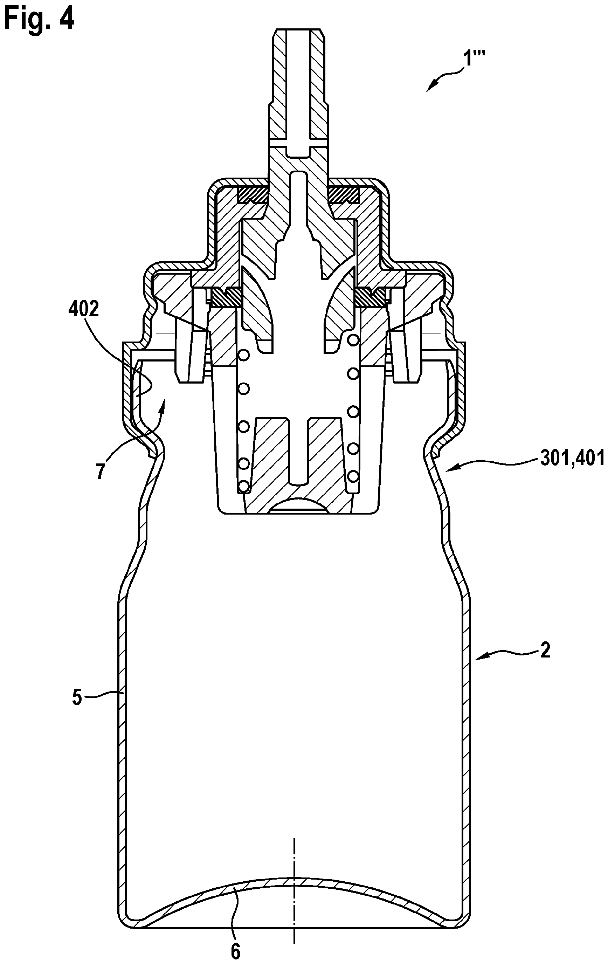

Figure 4 shows a fourth embodiment of a medicament canister 1 according to the present invention. The medicament canister 1‴ differs from the one shown in Figure 3 in that the reduced diameter section 301 is formed by a concave portion 401 of the sidewall 5 which connects an upper tubular sidewall portion 402 extending from the concave portion 401 to the open upper end 7 of the container 2 with the tubular sidewall 5 of the container 2. The tubular sidewall extends from the bottom wall 6 of the container 2 to the reduced diameter section 301. The tubular wall portion 10 of the cap housing 9 is deformed to abut the upper tubular sidewall portion 402 and the side wall 5 at the reduced diameter section 301.

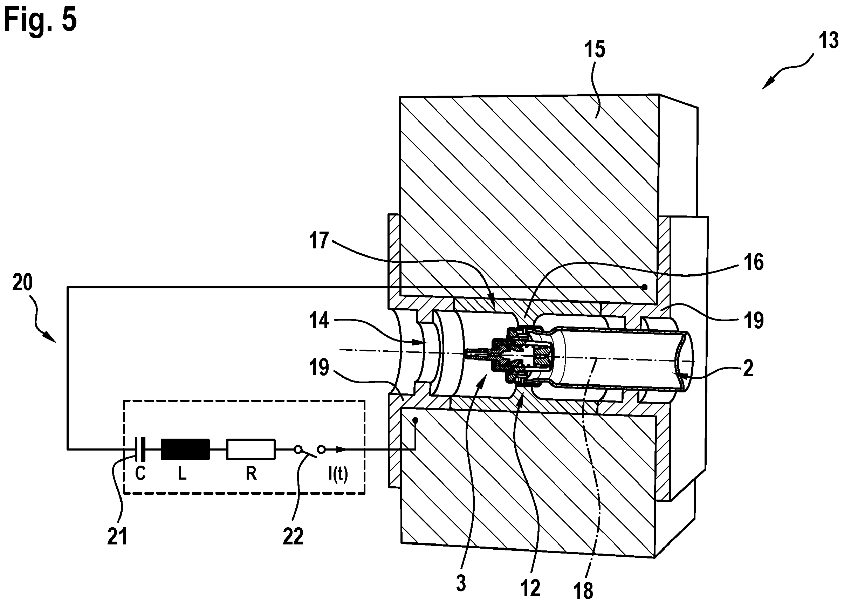

Figure 5 shows an apparatus 13 for producing the gas tight medicament canister 1, 1', 1'', 1''' as shown in Figures 1 and 2 to 4 for metered dose inhalers (not shown) by electromagnetic pulse welding or electromagnetic pulse crimping according to the present invention. The apparatus 13 comprises a receiving portion 14 configured to receive the container 2 and the valve cap 3.

The apparatus 13 is configured to generate one or more magnetic field pulses to the overlap region 12 between the lower end 11 of the cap housing 9 and the open upper end 7 of the container 2. The apparatus 13 comprises a coil 15 surrounding the overlap region 12 and a field shaper 16 positioned between the coil 15 and the overlap region 12. The field shaper 16 is isolated from the coil 15.

The field shaper 16 is designed as two parts each forming a half shell. The field shaper 16 has a cross sectional area 17 along its longitudinal axis 18, which tapers towards the overlap region 12 between the lower end 11 of the cap housing 9 and the open upper end 7 of the container 2. The field shaper 16 is configured to concentrate the magnetic pressure force generated by the one or more magnetic field pulses on the overlap region 12. The field shaper 16 and the coil 15 are arranged concentrically with regard to the longitudinal axis 18.

The apparatus 13 comprises a centering guide 19 adapted to center the container 2 and the valve cap 3 in the field shaper 16 and the coil 15. Accordingly, the container 2, and the valve cap 3 are also arranged concentrically with regard to the longitudinal axis 18.

The apparatus 13 further comprises a circuit 20 connected to the coil 15, wherein the circuit 20 comprises a capacitor 21 and a switch 22. Optionally, the capacitor 21 may be formed as a capacitor bank depending on the amount of energy needed to generate the one or more magnetic field pulses.

In the following a method for producing the gas tight medicament canister 1 for metered dose inhalers is explained with reference to Figures 1 and 2.

In a first and a second step the metal or plastic container 2 and the valve cap 3 are provided, respectively.

In a third step the container 2 and the valve cap 3 are inserted into the apparatus 13 which is configured to generate the one or more magnetic field pulses. The lower end 11 of the cap housing 9 is positioned over the upper end 7 of the container 2 such that at least a portion of the tubular wall portion 10 of the cap housing 9 overlaps with a portion of the circumferential side wall 5 of the container 2, thereby forming the overlap region. No gasket is inserted between the side wall 5 of the container 2 and the cap housing 9 of the valve cap 3.

In a subsequent step the capacitor 21 is charged by use of a power supply (not shown) with a predetermine amount of energy. The predetermined amount of energy to be stored in the capacitor 21 is at most 0,9 kJ, preferably at most 0,75 kJ, more preferably at most 0,45 kJ, most preferably at most 0,3 kJ. The amount of energy used defines whether there will be a substance-to-substance bond between the valve cap 3 and the container 2 or whether there will be a mechanical connection between the both. A substance-to-substance bond will be achieved by electromagnetic pulse welding. Using a lower predetermined amount of energy to be stored in the capacitor 21 will result in a mechanical connection between the valve cap 3 and the container 2 which is referred to in the present invention as electromagnetic pulse crimping. Besides the energy input further parameters such as the material combination of the parts to be connected, the wall thickness of the parts at the area to be connected, the distance between the parts at the area to be connected and the material properties may influence whether the parts to be connected are joined by electromagnetic pulse welding or electromagnetic pulse crimping.

Subsequently, the switch 22 is closed and the energy stored in the capacitor 21 is instantaneously released into the coil 15 surrounding the overlap region 12. This generates a magnetic field pulse to the overlap region 12, thereby generating a magnetic pressure force which acts in a direction substantially perpendicular to a circumference of the overlap region 12 and causes the portion of the tubular wall portion 10 of the cap housing 9 to impact onto the portion of the tubular sidewall 5 of the container 2. Thereby, the portion of the tubular wall portion 10 and the portion of the tubular side wall 5 are joined by electromagnetic pulse welding or electromagnetic pulse crimping. In case of electromagnetic pulse welding the magnetic pressure force is higher compared to the electromagnetic pulse crimping which results in a higher impact of the tubular wall portion 10 of the cap housing 9 onto the portion of the tubular sidewall 5 of the container 2 and a substance-to-substance bond between the both.

In detail: When instantaneously releasing the energy stored in the capacitor 21 into the coil 15, a generated discharge current induces a strong transient magnetic field inside the coil 15, thereby generating the magnetic field pulse. The magnetic field in turn induces eddy currents in the portion of the tubular wall portion 10 of the cap housing 9. These eddy currents prevent the magnetic field to diffuse through the tubular wall portion 10 of the cap housing 9 and cause a difference in magnitude of the magnetic field on the portion of the tubular wall portion 10 of the cap housing 9 and the portion of the side wall 5 of the container 2. The difference generates a magnetic pressure force, which causes the portion of the tubular wall portion 10 of the cap housing 9 to impact with the portion of the side wall 5 of the container 2. In case of electromagnetic pulse welding the collision between both portions causes bonding through several bonding mechanisms.

In a final step the obtained medicament canister 1 can be provided to a filling station (not shown). Therein, the canister 1 is filled with propellant and medicament under pressure via the metering valve 8. Alternatively, the medicament container (2) is filled with medicament prior to joining the container (2) and the cap housing (9) of the valve cap (3) and the canister (1) is filled with propellant thereafter via the metering valve (8).

### Reference Numerals

- 1, 1', 1", 1‴: Medicament canister
- 2: Metal or plastic container
- 3: Valve cap
- 4: Cavity
- 5: Tubular side wall
- 6: Bottom wall
- 7: Upper end (container)
- d1: First diameter (container)
- 8: Metering valve
- 9: Cap housing
- 10: Tubular wall portion (cap housing)
- 11: Lower end (cap housing)
- d2: Second diameter
- 12: Overlap region
- 13: Apparatus
- 14: Receiving portion
- 15: Coil
- 16: Field shaper
- 17: cross sectional area (field shaper)
- 18: Longitudinal axis (field shaper)
- 19: Centering guide
- 20: Circuit
- 21: Capacitor
- 22: Switch
- 100: medicament canister (prior art)
- 101: metal container (prior art)
- 102: upper end (metal container, prior art)
- 103: valve cap (prior art)
- 104: cap housing (prior art)
- 105: lower rend (cap housing, prior art)
- 106: gasket (prior art)
- 201: flange (second embodiment)
- 202: upper side (flange)
- 203: lower side (flange)
- 301: reduced diameter section (third and fourth embodiment)
- 302: concave portion (reduced diameter section)
- 401: concave portion (reduced diameter section)
- 402: upper tubular sidewall portion (fourth embodiment)

## Claims

1. A method for producing a medicament canister (1) for metered dose inhalers, the medicament canister (1) comprising a metal or plastic container (2) and a valve cap (3), wherein the method comprises the steps of:
a) providing the container (2) with an interior cavity (4) defined by a circumferential side wall (5) extending from a bottom wall (6) to an open upper circular end (7) of the container (2), the side wall (5) at the open upper end (7) of the container (2) having a first diameter (d1) ;
b) providing the valve cap (3) having a metering valve (8) and a cap housing (9), in which the metering valve (8) is mounted, the cap housing (9) having a tubular wall portion (10) forming an open lower end (11) of the cap housing (9), wherein the tubular wall portion (10) has a second diameter (d2) which is slightly larger than the first diameter (d1) at the open upper end (7) of the container (2), such that, upon overlap of the open lower end (11) of the cap housing (9) and the open upper end (7) of the container (2), the tubular wall portion (10) encloses the side wall (5) of the container (2);
c) positioning the lower end (11) of the cap housing (9) over the upper end (7) of the container (2) such that at least a portion of the tubular wall portion (10) of the cap housing (9) overlaps with a portion of the side wall (5) of the container (2), thereby forming an overlap region (12) between the lower end (11) of the cap housing (9) and the open upper end (7) of the container (2);
d) applying one or more magnetic field pulses to the overlap region (12) between the lower end (11) of the cap housing (9) and the open upper end (7) of the container (2), thereby generating a magnetic pressure force which acts on the portion of the tubular wall portion (10) of the cap housing (9) and/or the portion of the side wall (5) of the container (2) in a direction substantially perpendicular to a circumference of the overlap region (12), thereby joining the portion of the tubular wall portion (10) and the portion of the side wall (5) in a gas tight manner.

2. The method according to claim 1, wherein in step b) the difference between the second diameter (d2) of the tubular wall portion (10) and the first diameter (d1) at the open upper end (7) of the container (2) lies in the range of about 0.2 mm to 8 mm, preferably of about 1 mm to 5 mm, more preferably of about 1.5 mm to 3 mm, most preferably is about 2 mm.

3. The method according to claim 1, wherein in step a) the side wall extending from the bottom wall (6) to the open upper circular end (7) of the container (2) has a tubular shape and wherein in step c) at least a portion of the tubular wall portion (10) of the cap housing (9) overlaps with a portion of the tubular side wall (5) of the container (2) .

4. The method according to claim 3, wherein in step a) the tubular side wall comprises an annular flange at the open upper end (7) of the container (2), the flange extending in a radial outward direction and wherein in step c) at least a portion of the tubular wall portion (10) of the cap housing (9) overlaps with the flange and a portion of the tubular side wall (5) of the container (2).

5. The method according to any of claims 1 or 2, wherein in step a) the side wall comprises a reduced diameter section and wherein in step d) the tubular wall portion (10) of the cap housing (9) is deformed to abut the side wall at the reduced diameter section.

6. The method according to any of the preceding claims, wherein step c) includes the inserting of the container (2) and the valve cap (3) into an apparatus (13) configured to generate the one or more magnetic field pulses, the apparatus (13) comprising:
- a coil (15) surrounding the overlap region (12) between the lower end (11) of the cap housing (9) and the open upper end (7) of the container (2),
- a field shaper (16) positioned between the coil (15) and the overlap region (12), the field shaper (16) being configured to concentrate the magnetic pressure force on the overlap region (12), and
- a capacitor (21) connected to the coil (16).

7. The method according to claim 6, wherein the field shaper (16) is designed as two parts each forming a half shell.

8. The method according to claim 6, wherein the apparatus (13) further comprises a centering guide (19) adapted to center the container (2) and the valve cap (3) in the field shaper (16) and the coil (15).

9. The method according to claim 6, wherein step d) includes the charging of the capacitor (21) with a predetermine amount of energy, the instantaneously releasing of the energy into the coil (15) surrounding the overlap region (12), thereby generating the magnetic pressure force and causing the portion of the tubular wall portion (10) of the cap housing (9) to impact onto the portion of the tubular sidewall (5) of the container (2).

10. The method as claimed in claim 9, wherein the predetermined amount of energy stored in the capacitor (21) is at most 0,9 kJ, preferably at most 0,75 kJ, more preferably at most 0,45 kJ, most preferably at most 0,3 kJ.

11. The method according to any of the preceding claims, wherein no gasket is inserted between the tubular side wall (5) of the container (2) and the cap housing (9) of the valve cap (3).

12. The method according to any of the preceding claims, wherein the container (2) and/or the cap housing (9) are made of steel, stainless steel or aluminum.

13. The method according to any of the preceding claims, wherein the medicament canister (1) is filled with propellant and medicament under pressure via the metering valve (8) or wherein the medicament container (2) is filled with medicament prior to joining the container (2) and the cap housing (9) of the valve cap (3) and the canister (1) is filled with propellant thereafter via the metering valve (8).

14. Medicament canister for metered dose inhalers, wherein the medicament canister (1) is obtainable by the method according to claim 1, the medicament canister (1) comprising an annular gas tight connection between the container (2) and the valve cap (3) in the overlap region (12).

15. Medicament canister according to claim 14, **characterized in that** there is no gasket between the tubular side wall (5) of the container (2) and the cap housing (9) of the valve cap (3).
